# EUROPEAN PATENT SPECIFICATION

(11) **EP 3 897 964 B1**
(45) Date of publication and mention of the grant of the patent: **07.06.2023**
(21) Application number: 19828709.6
(22) Date of filing: 18.12.2019
(51) Int. Cl.: B01J 19/18, B01J 8/10, B01F 27/00

(54) **FLOW DISTRIBUTOR AND REACTOR USING SUCH FLOW DISTRIBUTOR**
STRÖMUNGSVERTEILER UND REAKTOR MIT EINEM SOLCHEN STRÖMUNGSVERTEILER
DISTRIBUTEUR D'ÉCOULEMENT ET RÉACTEUR UTILISANT UN TEL DISTRIBUTEUR D'ÉCOULEMENT

(30) Priority: 21.12.2018 SE 1851650
(43) Date of publication of application: 27.10.2021
(73) Proprietor: Spinchem AB, 907 36 Umeå (SE)
(72) Inventor: BYSTRÖM, Emil, 922 66 Tavelsjö (SE)
(74) Representative: AWA Sweden AB
(86) International application number: PCT/EP2019/085935
(87) International publication number: WO 2020/127472

(56) References cited:
- WO-A1-2017/182545
- WO-A2-03/033097
- CH-A5- 604 874

## Description

### Field of Invention

The present invention refers to a cylindrical flow distributor for performing, by means of solid reaction members, a biological or chemical transformation, or physical or chemical trapping from, or release of agents to, a fluidic media. The invention further refers to a reactor using a cylindrical flow distributor.

### Background of the invention

Heterogeneous processes in chemistry and biotechnology are unit operations that encompass a solid member (including, but not limited to, immobilized chemical reagents, catalysts, scavengers, reaction supports, trapping sorbents, or immobilized biological materials such as enzymes, or cells or fragments thereof) contacting a fluidic medium carrying reactants or other agents, sample solutes, and/or products of the interactive processing of fluid-conveyed agent(s) with the solid members. Most such heterogeneous processes are critically dependent on convective flow of the fluidic medium to establish the necessary mass transfer between the fluidic and solid phases.

Such systems are therefore often operated in a continuous flow through mode, in which case a conventional packed column with a suitable design is often the preferred format for encapsulating the solid member that is to be transited or percolated by the reaction medium. Numerous processes are, however, unfit for continuous processing. This applies in particular to processes where sequential addition of agents and/or removal of by-products or desired products are necessary, or where the physical or chemical conditions must otherwise be altered during the course of processing with the solid member. In those cases, a batchwise processing mode is often preferred.

One way is to use a flow distributor. This type of reactor can in be described as a rotary body which is filled with reaction members, immersed in a fluid and then set to rotate to thereby promote a chemical reaction.

To this date, all flow distributors have been manufactured by using a woven filter with an open mesh structure. Typically, a sheet having such open mesh structure is wrapped and welded to form a cylinder. The accuracy in the welding has to be in the order of 0.01-0.001 mm. The resulting cylinder is placed in a scaffold, e.g. an outer ring, to stabilize and support the welded filter mesh. Radial gaps caused by difference in diameters of the cylinder and the scaffold is a huge problem.

The goal is typically to provide a snug fit between the inner walls of the scaffold and the outer wall of the cylinder, however it is very difficult to meet this kind of small tolerances in production and especially since welding causes thermal expansion that may cause buckling of the woven filter wall of the cylinder.

There is hence a need for a new design of a flow distributor that allows a simplified flow distributor. WO2017/182545 discloses a cylindrical flow distributor to be used in a reactor.

### Summary of the invention

According to a first aspect, the invention provides a cylindrical flow distributor for performing, by means of solid reaction members, a biological or chemical transformation, or physical or chemical trapping from, or release of agents to, a fluidic media, the flow distributor comprising:
a top wall;
a bottom wall comprising a central through going opening; and
an outer wall extending between the top wall and the bottom wall;
wherein the top wall, the bottom wall and an inner envelope surface of said outer wall together define a confinement configured to contain solid reaction members or a rigid body of a reaction member material; and
wherein the outer wall comprises a first plurality of longitudinally extending ribs arranged side by side along a longitudinal centerline CL of the flow distributor with gaps extending in the circumferential direction between two adjacent ribs, and a circumferentially extending first scaffold encircling and being fixedly attached to a peripheral outer surface of said plurality of longitudinally extending ribs.

The cylindrical flow distributor further comprises an inner wall comprising a second scaffold having an extension along the longitudinal center line of the flow distributor, and a plurality of consecutive turns of a second wire being fixedly attached to an outer surface of said second scaffold, such that axially extending gaps are formed between each turn of said second wire, wherein the top wall, the bottom wall, the inner envelope surface of the outer wall and an outer envelope surface of the inner wall facing the outer wall together define the confinement configured to contain solid reaction members or a rigid body of a reaction member material.

Thereby a cylindrical flow distributor is provided in which a confinement is defined by the top wall, the bottom wall and the outer wall. Before use of the flow distributor, the confinement is provided with loosely arranged solid reaction members or one or more cartridges holding such solid reaction members, or a rigid body of a reaction member material (monolithic). In use, the flow distributor containing such solid reaction members is configured to be immersed in a fluid containing vessel and then subjected to a rotation around its own longitudinal centerline. As a result of the rotation, a vortex is generated which causes the fluid to be sucked into the confinement via the central through going opening in the bottom wall and then be forced in the radial direction by a centrifugal action, past the reaction members, before leaving the confinement via the longitudinal gaps that are formed between the first plurality of longitudinally extending ribs. The width of the gaps is adapted to the size of the reaction members to prevent the members from escaping there through.

By the outer wall being formed by a plurality of ribs supported by a scaffold, the manufacturing of the flow distributor is substantially facilitated as compared to prior art using a sheet of a woven filter with an open mesh structure which is wrapped and welded to form a cylinder. The arrangement with ribs and a scaffold comprises a larger inherent mass of the material which allows a better and more even heat distribution during welding. Thereby the risk of temperature related geometrical deformations is reduced. Any such deformations cause the risk of insufficient or even lack of contact surfaces during welding. Hence, the present solution presents a more rigid and more dimensional stable flow distributor.

Further, a flow distributor is provided that allows a substantially facilitated cleaning. The cleaning may e.g. be made by using a brush with bristles that are inserted into and guided along the gaps which are formed between the ribs. The longitudinal gaps eliminate any problems with particulate matter getting entangled in a fine mesh structure. Thus the difficulty of cleaning a fine meshed wall structure without breaking the same or its joints is overcome. Hence, also a potentially longer life time of the flow distributor is provided for.

Additionally, and not at least, the now presented cylindrical flow distributor has shown to provide a substantially reduced time to complete a chemical reaction. Trials have shown that the time to complete a chemical reaction may be reduced with more than 30 % than using a prior art flow distributor having walls of a mesh structure. The provision of longitudinally extending gaps is considered to present a reduced flow resistance for the fluidic flow during high speed rotation as compared to a fine mesh structure in which the boundaries of each hole in the mesh presents a flow restriction. Thereby a faster throughput of the fluidic medium is allowed.

The first scaffold may be a first wire spiral wound around the peripheral outer surface of said first plurality of longitudinally extending ribs. The purpose of the first scaffold is to fixate the ribs to provide a robust and dimensional stable outer wall structure to the flow distributor. The skilled person will understand that the pitch of the turns of the first wire should be adapted to the diameter and also the height of the cylindrical flow distributor. It is preferred that the pitch is adapted so that there is at least three and preferably more fixation points between each rib and the first wire. However, the pitch of the spiral wound first wire should be adapted so that it does not unduly interfere with the radial fluid flow through the gaps to be established during use of the flow distributor.

The gaps extending between two adjacent ribs may have a width W_{A} as measured along the inner envelope surface of said confinement being smaller than the size of solid reaction members or a rigid body of a reaction member material (monolithic) to be contained in the confinement. The gaps extending between two adjacent ribs may have a width of 10-1000 µm, and more preferred 20-800 µm and even more preferred 20-500 µm as measured along the inner envelope surface of said confinement. The width should be small enough to prevent solid reaction members or a rigid body of a reaction member material (monolithic) configured to be contained in the confinement from being released there through. Still, the width must be large enough from not unduly restricting a fluid flow there through.

Each rib in the first plurality of ribs may have a triangular or trapezoid cross section, and wherein each rib may be fixedly attached to an inner surface of the first scaffold via its apex in the event of the cross section being triangular or via its shortest parallel edge in the event of the cross section being trapezoid, whereby the gaps extending between two adjacent ribs as measured along the inner envelope surface of said outer wall is smaller than the gaps extending between two adjacent ribs as measured along an outer envelope surface of said outer wall.

Accordingly, the inner envelope surface of the outer wall will be formed by the bases of the plurality of ribs in the event the rib's cross sections being triangular and by the long parallel edges in the event the rib's cross sections being trapezoid. No matter cross section, the inner envelope surfaces of the outer wall will be cylindrical. Since the ribs are typically made from an extruded, cut wire, the skilled person will understand that production tolerances may hinder the edge portions, no matter cross section, from being completely straight.

The attachment between the second wire and the second scaffold is preferably made by welding. However, it is to be understood that also other methods may be used, such as adhesive bonding or even brazing.

The gaps between each turn of said second wire may have a width W_{B} as measured in the axial direction along the outer envelope surface of said inner wall being smaller than the size of solid reaction members or a rigid body of a reaction member material (monolithic) to be contained in the confinement. The gaps between each turn of said second wire may have a width of 10-1000 µm and more preferred 20-800 µm and even more preferred 20-500 µm as measured in the axial direction along the outer envelope surface of said inner wall. The width should be small enough to prevent solid reaction members or a rigid body of a reaction member material (monolithic) configured to be contained in the confinement from being released there through. Still, the width must be large enough from unduly restricting a fluid flow there through.

The second wire may have a triangular or trapezoid cross section, and the second wire may be fixedly attached to an outer surface of the second scaffold via its apex in the event of the cross section being triangular or via its shortest parallel edge in the event of the cross section being trapezoid, whereby the gaps extending between two adjacent turns of the second wire as measured along the outer envelope surface of said inner wall facing the outer wall is smaller than the gaps extending between two adjacent turns of the second wire as measured along an inner envelope surface of said inner wall facing away from the outer wall.

Accordingly, the outer envelope surface of the inner wall of the flow distributor will be formed by the base of the second wire in the event the wire having a triangular cross section and by the long parallel edge in the event the wire having a trapezoid cross section. No matter cross section, the outer envelope surfaces of the inner wall will be cylindrical. Since the wire typically is extruded, the skilled person will understand that production tolerances may hinder the edge portions no matter cross section from being completely straight.

The second scaffold may comprise a second plurality of axially extending ribs. One purpose of the second scaffold is to fixate the second wire to thereby provide a robust inner wall structure to the flow distributor. The plurality of ribs may be arranged side by side with gaps extending in the circumferential direction between two adjacent ribs. The number of ribs should be adapted so that it does not unduly interfere with the radial fluid flow through the gaps to be established during use of the flow distributor

The top wall may be configured to support a shaft, said shaft being configured to be connected to a means, such as a motor, for rotating the cylindrical flow distributor. It is to be understood that other positions of the shaft are possible.

At least one of the top wall or the bottom wall may be removably connected to the outer wall or to the inner wall. Thereby the flow distributor may be easily disassembled to allow loading and unloading of the confinement with solid reaction members or a rigid body of a reaction member material, but also to facilitate cleaning of the confinement.

The cylindrical flow distributer may further comprise radially extending partition walls. The partition walls may be arranged as a unitary body forming a removable arranged insert. The partition walls may be useful to restrict movement of the solid reaction members or one or more rigid bodies of a reaction member material inside the cylindrical flow distributor during use thereof. Thereby, the life length of the reaction members may be increased and also cleaning of the cylindrical flow distributer may be facilitated. The partition walls may further be arranged to hold fluid permeable cartridges filled with solid reaction members in place.

According to another aspect, a reactor for performing, by means of solid reaction members, a biological or chemical transformation, or physical or chemical trapping from, or release of agents to, a fluidic media, is provided. The reactor comprises a vessel in which a cylindrical flow distributor has been mounted, said cylindrical flow distributor comprising:
a top wall;
a bottom wall comprising a central through going opening;
an outer wall extending between the top wall and the bottom wall; and
a means for rotating the cylindrical flow distributor;
wherein the top wall, the bottom wall and an inner envelope surface of said outer wall together define a confinement configured to contain solid reaction members or a rigid body of a reaction member material;
wherein the outer wall comprises a first plurality of longitudinally extending ribs arranged side by side along a longitudinal centerline CL of the flow distributor with gaps extending in the circumferential direction between two adjacent ribs, and a circumferentially extending first scaffold encircling and being fixedly attached to a peripheral outer surface of said plurality of longitudinally extending ribs.

The reactor further comprises an inner wall, wherein the top wall, the bottom wall, the inner envelope surface of the outer wall and an outer envelope surface of the inner wall facing the outer wall together define the confinement configured to contain solid reaction members or a rigid body of a reaction member material; and
wherein the inner wall comprises a second scaffold having an extension along an axial direction of the flow distributor, and a plurality of consecutive turns of a second wire being fixedly attached to an outer surface of said second scaffold, such that axially extending gaps are formed between each turn of said second wire.

The cylindrical flow distributor as such with its design, advantages and operation has been discussed above. To avoid undue repetition, reference is made to the sections above which are directly applicable also to a rector comprising such cylindrical flow distributor.

### Brief description of the enclosed figures

The present invention will be further disclosed with reference to the enclosed figures, in which:
Fig. 1 discloses a perspective view from above of one embodiment of a cylindrical flow distributor.
Fig. 2 discloses a perspective view from below of one embodiment of a cylindrical flow distributor.
Fig. 3 discloses highly schematically a cross section of the cylindrical flow distributor as seen along the longitudinal centerline.
Fig. 4 discloses highly schematically a cross section of the confinement of the cylindrical flow distributor as seen transverse its longitudinal centerline.
Fig. 5A and 45B discloses highly schematically a longitudinal and a transversal cross section of the outer wall.
Fig. 6A and 6B discloses highly schematically a longitudinal and a transversal cross section of the inner wall.
Fig. 7 discloses highly schematically one embodiment of a reactor.
Fig. 8 is a graph disclosing a comparison between a flow distributor according to the present invention and a flow distributor according to prior art.

### Detailed description of preferred embodiments

In the following one embodiment of the present invention will be discussed starting with the overall design of the cylindrical flow distributor.

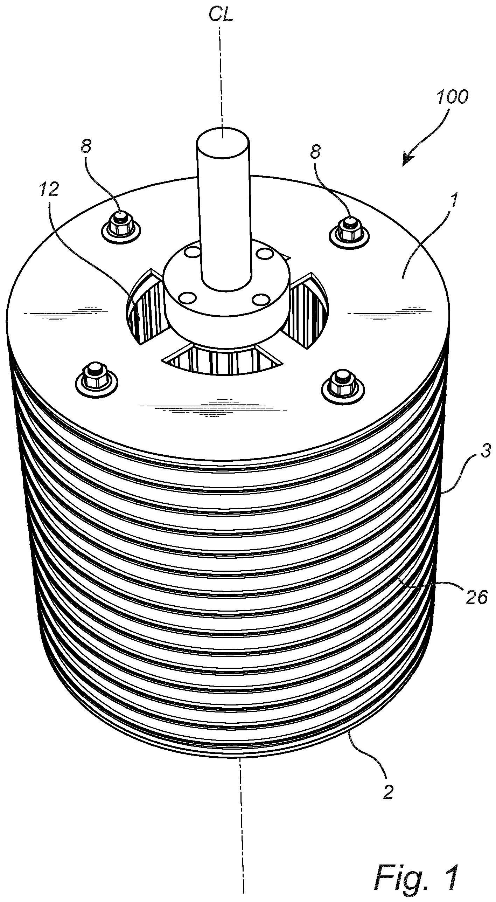

Starting with Figs. 1-4 two perspective views, a transverse cross sectional view and a longitudinal cross sectional view of one embodiment of the flow distributor 100 are disclosed. To facilitate the disclosure, the inner and outer walls are partly schematically illustrated as solid surfaces in these views.

The cylindrical flow distributor 100 comprises a top wall 1, a bottom wall 2, an outer wall 3 and an inner wall 4. The outer wall 3 extends between the top wall 1 and the bottom wall 2 and comprises an inner envelope surface 5 facing the longitudinal center line CL of the flow distributor 100. The inner wall 4 extends between the top wall 1 and the bottom wall 2 and comprises an outer envelope surface 6 facing the outer wall 3. The top wall 1, the bottom wall 2, the inner envelope surface 5 of the outer wall 3, and the outer envelope surface 6 of the inner wall 4 do together define a confinement 7 configured to contain solid reaction members (not disclosed) or a rigid body of a reaction member material (monolithic).

Although the flow distributor 100 in the following will be described and illustrated with the inner wall 4, it is to be understood that the flow distributor 100 with remained function may be provided without the inner wall 4, whereby the confinement 7 instead will be defined by the top wall 1, the bottom wall 2 and the inner envelope surface 5 of the outer wall 3 only.

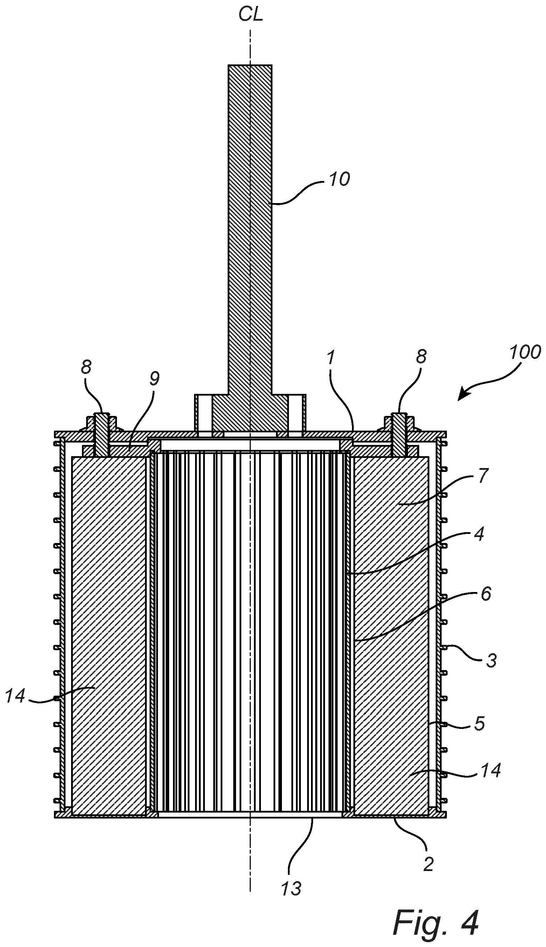

As is best seen in Fig. 4, the top wall 1 forming a lid is removable attached by a plurality of screws 8 to a radially extending flange 9 that projects from the inner wall 4. It is to be understood that the flange 9 with remained function may extend from the outer wall 3. It is to be understood that the top wall 1 may be mounted in numerous ways of less relevance to the invention.

The top wall 1 supports a shaft 10. The shaft 10 is configured to be connected to a means, such as a motor, for rotating the flow distributor 100. It is to be understood that other positions of the shaft are possible with remained function.

In the disclosed embodiment, the top wall 1 comprises a central through going opening 12. This opening may be omitted with remained function.

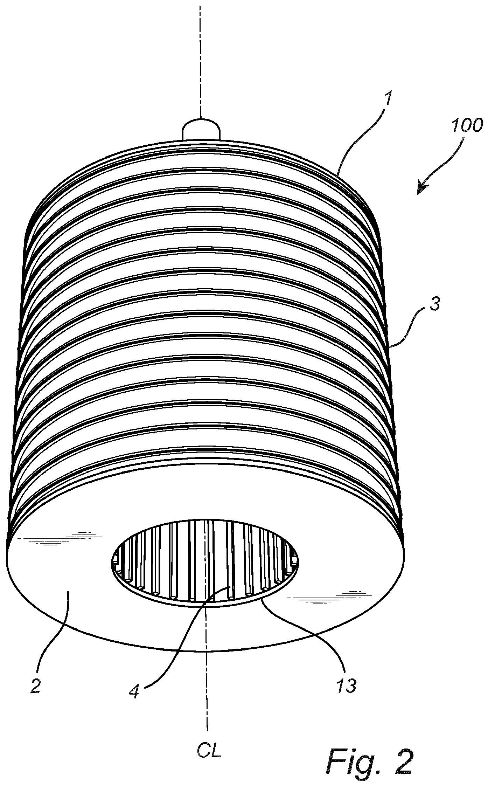

As is best seen in Figs. 2 and 4, the bottom wall 2 has a central throughgoing opening 13 which is arranged in the vicinity of the center of the bottom wall 2. The cross sectional area of the opening 13 is substantially smaller than the cross sectional area of the bottom wall 2.

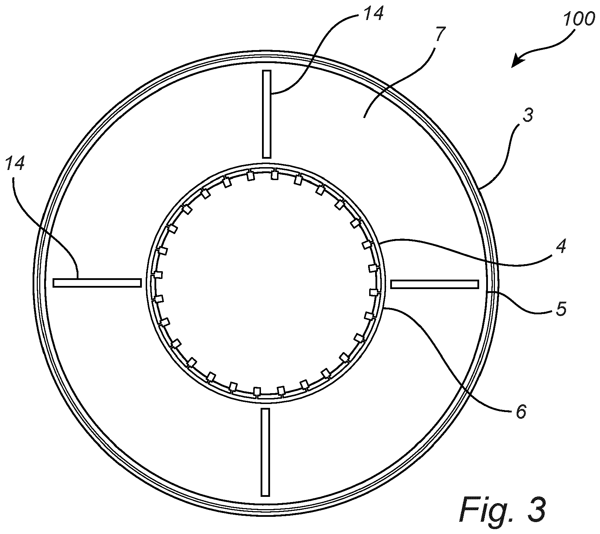

As is best seen in Figs. 3 and 4, the confinement 7 contains a plurality of radially extending partition walls 14. The partition walls 14 are attached to the radially extending flange 9. It is to be understood that the partition walls 14 may be arranged or supported in other ways, e.g. by the inner wall 4 or by the outer wall 3. The partition walls 14 may be arranged as a removable insert. The partition walls 14 may be useful to restrict movement of the solid reaction members or one or more rigid bodies of a reaction member material (monolithic) inside the cylindrical flow distributor 100 during use thereof. Thereby, the life length of the reaction members (not disclosed) may be increased and also cleaning of the cylindrical flow distributer 100 may be facilitated. The partition walls 14 may further be arranged to hold fluid permeable cartridges (not disclosed) filled with solid reaction members in place.

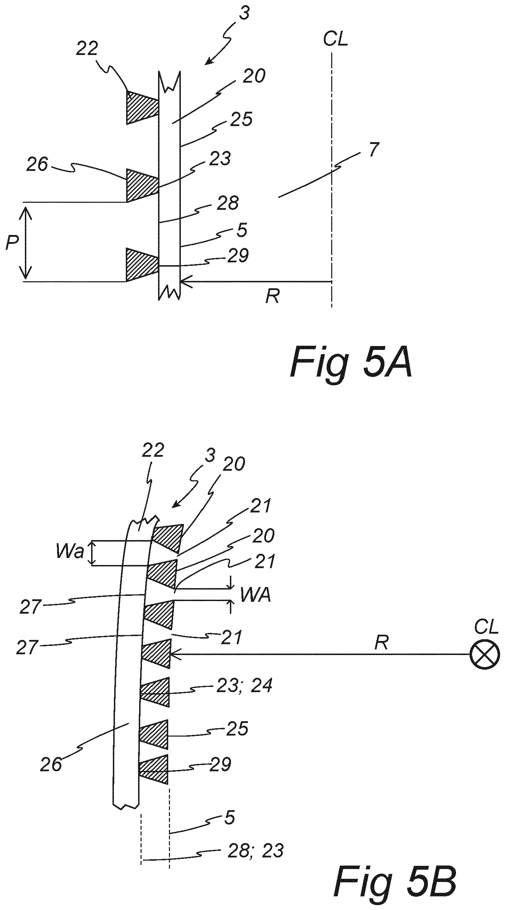

Now turning to Figs 1, 5A and 5B, the outer wall 3 will be discussed. Fig. 5A discloses highly schematically a cross section of the outer wall 3 as seen along the longitudinal centerline CL. Fig. 5B discloses highly schematically a portion of a cross section of the outer wall 3 transverse the longitudinal centerline CL.

The outer wall 3 comprises a first plurality of longitudinally extending ribs 20 arranged side by side along a longitudinal centerline CL of the flow distributor with gaps 21 extending in the circumferential direction between two adjacent ribs 20. The gaps 21 have a width W_{A} between two adjacent ribs 20 as measured along the inner envelope surface 5 of the confinement 7. The longitudinal gaps 21 are thus distributed in the circumferential direction.

A circumferentially extending first scaffold 22 to be discussed below encircles the ribs 20.

In the disclosed embodiment, each rib 20 has a trapezoid cross section. Each rib 20 is fixedly attached to an inner surface 23 of the first scaffold 22 via its shortest parallel edge 24. Thereby the width W_{A} of the gaps 21 extending between two adjacent ribs 20 as measured along the inner envelope surface 5 of said confinement 7 is smaller than the width Wₐ of the gaps 27 extending between two adjacent ribs 20 as measured along an outer envelope surface 28 of said outer wall 3. Said outer envelope surface 28 is defined by the inner surface 23 of the first scaffold 22.

The gaps 21 extending between two adjacent ribs 20 may have a width W_{A} as measured along the inner envelope surface 5 of said confinement 7 being smaller than the size of solid reaction members or a rigid body of a reaction member material (monolithic) to be contained in the confinement 7. The gaps 21 extending between two adjacent ribs 20 may have a width of 10-1000 µm, and more preferred 20-800 µm and even more preferred 20-500 µm as measured along the inner envelope surface 5 of said confinement 7. The width W_{A} should be small enough to prevent solid reaction members or a rigid body of a reaction member material (monolithic) configured to be contained in the confinement 7 from being released there through. Still, the width must be large enough from not unduly restricting a fluid flow there through.

It is to be understood that the ribs 20 may have other cross sections with remained function. By way of example, the cross section may be triangular. In that event, each rib 20 is fixedly attached to the inner surface 23 of the first scaffold 22 via its apex.

Accordingly, the inner envelope surface 5 of the confinement 7 will be formed by the bases of the plurality of ribs 20 in the event the rib's cross sections being triangular and by the long parallel edges 25 in the event the rib's cross sections being trapezoid. No matter cross section, the inner envelope surface 5 of the outer wall 3 will be substantially cylindrical. Since the ribs 20 are typically made from an extruded, cut wire, the skilled person will understand that production tolerances may hinder the edge portions no matter cross section from being completely straight.

The circumferentially extending first scaffold 22 encircles the ribs 20 and is fixedly attached to a peripheral outer surface 29 of said ribs 20. The purpose of the first scaffold 22 is to fixate the ribs 20 to provide a robust outer wall 3 structure to the flow distributor 100. The fixation is preferably made by welding. It is to be understood that other ways of fixation are possible, e.g. by using and adhesive or even brazing.

In the disclosed embodiment, the first scaffold 22 is formed by a first wire 26 that is spiral wound around the peripheral outer surface 29 of the ribs 20. It is preferred that the pitch P of the spiral is adapted so that there is at least three contact points, each forming a fixation point between each rib 20 and the first scaffold 22. However, the pitch P of the spiral wound first wire 26 should be adapted so that it does not unduly interfere with the radial fluid flow through the gaps 21 to be established during use of the flow distributor 100. The skilled person will understand that the pitch P should be adapted to the diameter and also the height of the cylindrical flow distributor 100.

In the disclosed embodiment the first wire 26 is disclosed as having a trapezoid cross section. It is to be understood that other cross sections are possible with remained function.

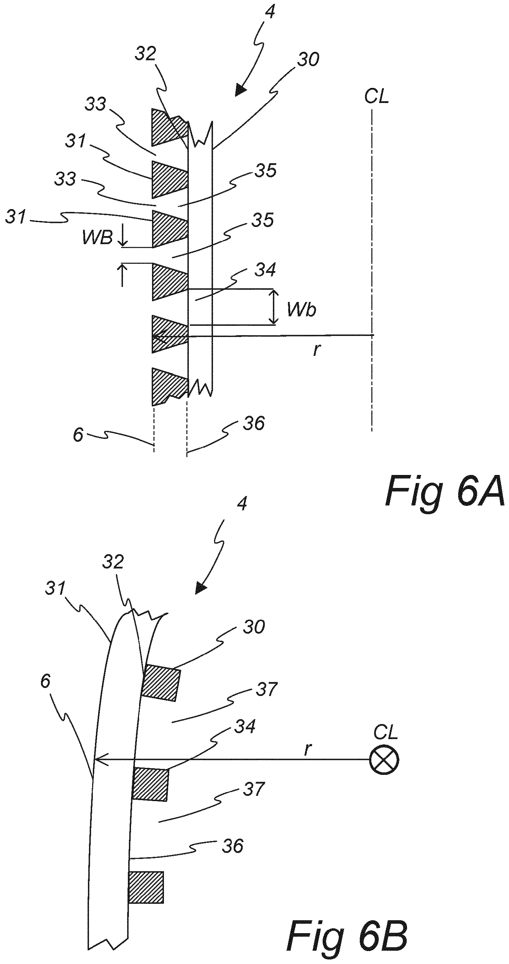

Now turning to Figs 1, 6A and 6B, the inner wall 4 will be discussed. Fig. 6A discloses highly schematically a cross section of the inner wall 4 as seen along the longitudinal centerline CL. Fig. 6B discloses highly schematically a portion of a cross section of the inner wall 4 transverse the longitudinal centerline CL.

The inner wall 4 comprises a second scaffold 30 having an extension along the axial direction of the flow distributor 100, i.e. along the longitudinal centerline CL.

The inner wall 4 comprises a plurality of consecutive turns of a second wire 31 being fixedly attached to an outer surface 32 of said second scaffold 30, such that axially extending gaps 33 are formed between each turn of the second wire 31.

The second wire 31 has in the disclosed embodiment a trapezoid cross section. The wire 31 is fixedly attached to the outer surface 32 of the second scaffold 30 via its shortest parallel edge, whereby the gaps 33 extending between two adjacent turns of the second wire 31 as measured along the outer envelope surface 6 of said inner wall 4 facing the outer wall 3 have a width W_{B} that is smaller than the width W_{b} of gaps 35 extending between two adjacent turns of the second wire 31 as measured along an inner envelope surface 36 of said inner wall 4.

The axially gaps 33 between each turn of said second wire 31 may have a width W_{B} as measured in the axial direction along the outer envelope surface 6 of said inner wall 4 being smaller than the size of solid reaction members or a rigid body of a reaction member material (monolithic) to be contained in the confinement. The gaps 33 between each turn of said second wire 31 may have a width W_{B} of 10-1000 µm and more preferred 20-800 µm and even more preferred 20-500 µm as measured in the axial direction along the outer envelope surface 6 of said inner wall 4. The width W_{B} should be small enough to prevent solid reaction members or a rigid body of a reaction member material (monolithic) configured to be contained in the confinement from being released there through. Still, the width W_{B} must be large enough from unduly restricting a fluid flow there through.

The second scaffold 30 comprises a second plurality of axially extending ribs 34. The second scaffold 30 is configured to fixate the second wire 31 to provide a robust inner wall 4 structure to the flow distributor 100. The plurality of ribs 34 may be arranged side by side with circumferentially distributed gaps 37 between two adjacent ribs 34. The number of ribs 34 should be adapted so that they do not unduly interfere with the radial fluid flow to take place through the gaps 33 along the along the outer envelope surface 6 of said inner wall 4. In the disclosed embodiment, the ribs 34 are disclosed as having a quadrangular cross section. It is to be understood that other cross sections are possible, such as a triangular or trapezoid cross section.

The attachment between the ribs 34 of the second scaffold 30 and the second wire 31 is preferably made by welding. It is to be understood that also other methods may be used, such as adhesive bonding or even brazing.

It is to be understood that the second wire 31 may have other cross sections with remained function. By way of example, the cross section may be triangular. In that event, the wire 31 is fixedly attached to the second scaffold 30 via its apex.

Accordingly, the inner envelope surface 6 of the inner wall 4, i.e. the envelope surface facing the outer wall 3 will be formed by the long parallel edge of the wire 31 in the event the second wire 31 having a trapezoid cross section and by the base of the wire 31 in the event the second wire 31 having a triangular cross section. No matter cross section, the inner envelope surface 6 of the inner wall 4 will be cylindrical. Since the second wire 31 typically is made from an extruded wire, the skilled person will understand that production tolerances may hinder the edge portions no matter cross section from being completely straight.

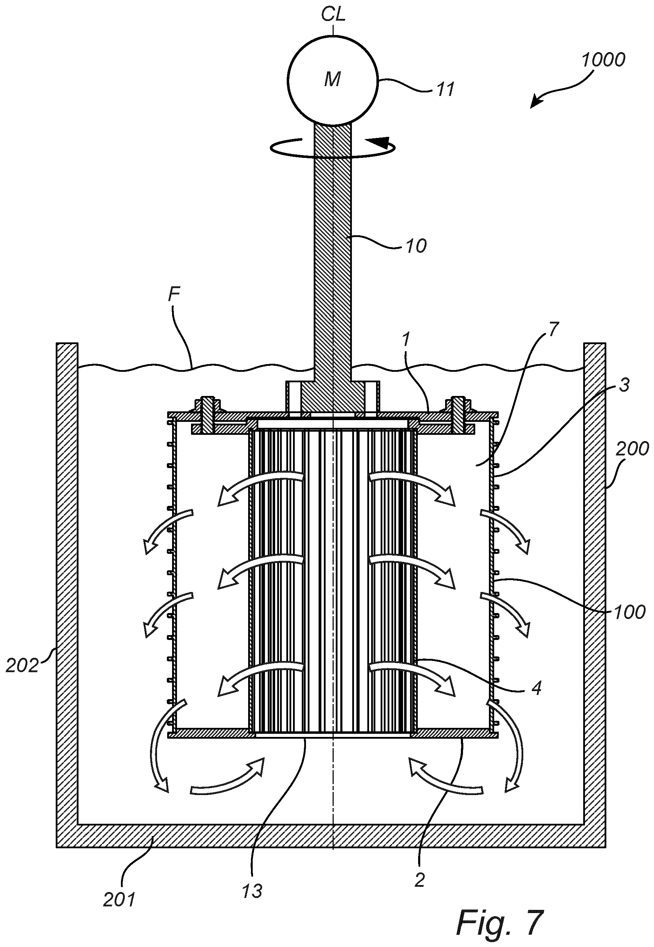

Reference is now made to Fig. 7 a reactor 1000 for performing, by means of reaction members, a biological or chemical transformation, or physical or chemical trapping from, or release of agents to, a fluidic media is schematically disclosed. The reactor 1000 comprises a vessel 200 in which a cylindrical flow distributor 100 has been mounted. The flow distributor 100 has the very same design as that previously disclosed, whereby like reference numbers are used for like features. To facilitate the disclosure, the ribs forming part of the outer wall 3 and the gaps between the ribs have been omitted. Also the partition wall has been omitted.

In the disclosed embodiment, the vessel 200 comprises a bottom wall 201 and a circumferentially extending side wall 202. It is to be understood that the vessel 200 by way of example also may be provided with non-disclosed features such as a lid and one or more valves. Further, vessel 200 may be provided with non-disclosed means for enhancing the fluidic shear stress in any of two rotary directions. The means for enhancing the fluidic shear stress may either be formed as a removable insert or be integral with the inner wall 4 of the vessel.

The flow distributor 100 is arranged to be operated by an electrically, pneumatically, or hydraulically driven motor 11 which is connected to the shaft 10 of the flow distributor 100.

In the following the operation of the flow distributor 100 will be discussed with reference to Figs. 7, 5A, 5B and 6A, 6B. The fluid flow to be described below is illustrated with arrows. As given above, the flow distributor 100 comprises a confinement 7 which is defined by the top wall 1, the bottom wall 2, the outer wall 3 and the optional inner wall 4. Before use of the flow distributor 100, the confinement 7 is loaded with loosely arranged solid reaction members (not disclosed), or one or more rigid bodies of a reaction member material (monolithic) or one or more cartridges (not disclosed) holding such reaction members. The thus loaded flow distributor 100 is immersed in a fluid F contained in the vessel 200 and connected to the motor 11 via its shaft 10. The motor 11 is activated whereby the flow distributor 100 is subjected to a high-speed rotation around its own longitudinal centerline CL. As a result of the rotation, a vortex is generated in the bottom of the vessel 200 which causes the fluid F to be sucked into the confinement 7 via the central through going opening 13 in the bottom wall 2 and then forced in the radial direction by a centrifugal action, past the solid reaction members, before leaving the confinement 7 via the gaps 21 that are formed between the first plurality of longitudinally extending ribs 20 in the outer wall 3. The width W_{A} of the gaps 21 is adapted to the size of the solid reaction members to prevent the members from escaping there through. In the event the flow distributor 100 also comprises an inner wall 4, the fluid will pass the gaps 35; 33 between the turns of the second wire 31 making up the inner wall 4 on its way into the confinement 7.

The rotation is maintained until the reaction has reached a target level. Then the rotation is stopped and the flow distributor 100 is removed from the vessel 200. The top wall 1 is removed and the reaction members are removed. Further, the flow distributor 100 is cleaned. The cleaning may be made by arranging the flow distributor in a vessel 200 comprising one or more nozzles configured to direct a cleaning fluid towards the exterior walls of the flow distributor while rotating the same. Alternatively, or supplementary, the cleaning may be made by using a brush.

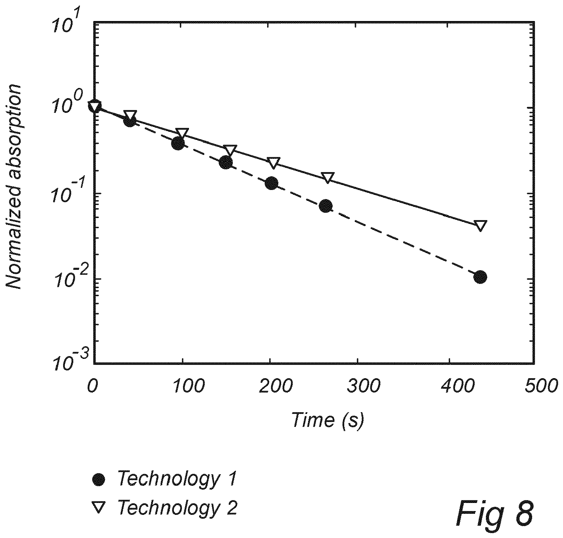

Now turning to Fig. 8 a comparison between the use of a flow distributor according to the invention and a prior art flow distributor comprising a confinement defined by finely meshed walls is disclosed. The dimensions of the two flow distributors are in all other aspects the same. As can be seen in the diagram, the efficiency, measured as normalized absorption, is about 30% better than the prior art tested flow distributor. The provision of longitudinally extending gaps 21; 33; 35 is considered to present a reduced flow resistance for the fluidic flow during high speed rotation as compared to a fine mesh structure in which the boundaries of each hole in the mesh presents a flow restriction.

The person skilled in the art realizes that the present invention by no means is limited to the preferred embodiments described above. On the contrary, many modifications and variations are possible within the scope of the appended claims.

As given above, in its easiest form, the flow distributor 100 may be arranged without the inner wall 4. In such embodiment, the confinement 7 will instead be defined by the top wall 1, the bottom wall 2 and the inner envelope surface 5 of the outer wall 3. In such embodiment, it is preferred that the solid reaction members are provided in cartridges to be received in the confinement 7 to thereby prevent them from accidentally falling out.

The first scaffold 22 has been described as a first wire 26 that is spiral wound around the peripheral outer surface 29 of said first plurality of longitudinally extending ribs 20. The wire 26 may by way of example be replaced by two or more rings encircling the ribs and which rings are distributed along the axial extension of the flow distributor.

## Claims

1. A cylindrical flow distributor for performing, by means of solid reaction members, a biological or chemical transformation, or physical or chemical trapping from, or release of agents to, a fluidic media, the flow distributor (100) comprising:
a top wall (1);
a bottom wall (2) comprising a central through going opening (13);
an outer wall (3) extending between the top wall (1) and the bottom wall (2); and
an inner wall (4);
wherein the outer wall (3) comprises a first plurality of longitudinally extending ribs (20) arranged side by side along a longitudinal centerline CL of the flow distributor (100) with gaps (21) extending in the circumferential direction between two adjacent ribs (20), and a circumferentially extending first scaffold (22) encircling and being fixedly attached to a peripheral outer surface (29) of said plurality of longitudinally extending ribs (20);
wherein the inner wall (4) comprises a second scaffold (30) having an extension along an axial direction of the flow distributor (100), and a plurality of consecutive turns of a second wire (31) being fixedly attached to an outer surface (32) of said second scaffold (30), such that axially extending gaps (33) are formed between each turn of said second wire (31); and
wherein the top wall (1), the bottom wall (2), an inner envelope surface (5) of the outer wall (3) and an outer envelope surface (6) of the inner wall (4) facing the outer wall (3) together define a confinement (7) configured to contain solid reaction members or a rigid body of a reaction member material.

2. A cylindrical flow distributor according to claim 1, wherein the first scaffold (22) is a first wire (26) spiral wound around the peripheral outer surface (29) of said first plurality of longitudinally extending ribs (20).

3. A cylindrical flow distributor according to claim 1 or 2, wherein the gaps (21) extending between two adjacent ribs (20) have a width W_{A} as measured along the inner envelope surface of said confinement (7) being smaller than the size of solid reaction members or the rigid body of a reaction member material to be contained in the confinement (7).

4. A cylindrical flow distributor according to any of the preceding claims, wherein each rib (20) in the first plurality of ribs (20) has a triangular or trapezoid cross section, and wherein each rib (20) is fixedly attached to an inner surface of the first scaffold (22) via its apex in the event of the cross section being triangular or via its shortest parallel edge in the event of the cross section being trapezoid, whereby the gaps (21) extending between two adjacent ribs (20) as measured along the inner envelope surface (5) of said outer wall (3) is smaller than the gaps (27) extending between two adjacent ribs (20) as measured along an outer envelope surface (28) of said outer wall (3).

5. A cylindrical flow distributor according to claim 1, wherein the gaps (33) between each turn of said second wire (31) have a width as measured in the axial direction along the outer envelope surface (6) of said inner wall (4) being smaller than the size of solid reaction members or the rigid body of a reaction member material to be contained in the confinement (7).

6. A cylindrical flow distributor according to any of the preceding claims, wherein the second wire (31) has a triangular or trapezoid cross section, and wherein the second wire (31) is fixedly attached to the outer surface (32) of the second scaffold (30) via its apex in the event of the cross section being triangular or via its shortest parallel edge in the event of the cross section being trapezoid, whereby the gaps (33) extending between two adjacent turns of the second wire (31) as measured along the outer envelope surface (6) of said inner wall (4) facing the outer wall (3) is smaller than gaps (35) extending between two adjacent turns of the second wire (31) as measured along an inner envelope surface (36) of said inner wall (4).

7. A cylindrical flow distributor according to any of claims 4-6, wherein the second scaffold (30) comprises a second plurality of axially extending ribs (34).

8. A reactor for performing, by means of solid reaction members, a biological or chemical transformation, or physical or chemical trapping from, or release of agents to, a fluidic media, said reactor comprising a vessel (200) in which a cylindrical flow distributor (100) has been mounted, said
cylindrical flow distributor (100) comprising:
a top wall (1);
a bottom wall (2) comprising a central through going opening (13);
an outer wall (3) extending between the top wall (1) and the bottom wall (2);
an inner wall (4); and
a means for rotating the cylindrical flow distributor (100);
wherein the outer wall (3) comprises a first plurality of longitudinally extending ribs (20) arranged side by side along a longitudinal centerline CL of the flow distributor with gaps (21) extending in the circumferential direction between two adjacent ribs (20), and a circumferentially extending first scaffold (22) encircling and being fixedly attached to a peripheral outer surface (29) of said plurality of longitudinally extending ribs (20),
wherein the inner wall (4) comprises a second scaffold (30) having an extension along an axial direction of the flow distributor (100), and a plurality of consecutive turns of a second wire (31) being fixedly attached to an outer surface (32) of said second scaffold (30), such that axially extending gaps (33) are formed between each turn of said second wire (31); and
wherein the top wall (1), the bottom wall (2), an inner envelope surface (5) of the outer wall (3) and an outer envelope surface (6) of the inner wall (4) facing the outer wall (3) together define a confinement (7) configured to contain solid reaction members or a rigid body of a reaction member material.

9. Use of a reactor according to claim 8 for performing by means of solid reaction members, a biological or chemical transformation, or physical or chemical trapping from, or release of agents to, a fluidic media.

## Patentansprüche

1. Zylindrischer Strömungsverteiler zum Durchführen, mittels fester Reaktionselemente, einer biologischen oder chemischen Transformation oder eines physikalischen oder chemischen Einfangens aus oder Freisetzens von Agenzien in ein fluidförmiges Medium, wobei der Strömungsverteiler (100) Folgendes umfasst:
eine obere Wand (1);
eine untere Wand (2), die eine mittige durchgehende Öffnung (13) umfasst;
eine Außenwand (3), die sich zwischen der oberen Wand (1) und der unteren Wand (2) erstreckt; und
eine Innenwand (4);
wobei die Außenwand (3) eine erste Mehrzahl von sich in Längsrichtung erstreckenden Rippen (20), die nebeneinander entlang einer Längsmittellinie CL des Strömungsverteilers (100) angeordnet sind, wobei sich Spalte (21) in der Umfangsrichtung zwischen zwei benachbarten Rippen (20) erstrecken, und ein sich in Umfangsrichtung erstreckendes erstes Gerüst (22) umfasst, das eine periphere Außenfläche (29) der Mehrzahl von sich in Längsrichtung erstreckenden Rippen (20) umgibt und daran fest angebracht ist;
wobei die Innenwand (4) ein zweites Gerüst (30), das eine Erstreckung entlang einer axialen Richtung des Strömungsverteilers (100) aufweist, und eine Mehrzahl von aufeinanderfolgenden Windungen eines zweiten Drahts (31) umfasst, der an einer Außenfläche (32) des zweiten Gerüsts (30) derart angebracht ist, dass sich axial erstreckende Spalte (33) zwischen jeder Windung des zweiten Drahts (31) gebildet sind; und
wobei die obere Wand (1), die untere Wand (2), eine innere Hüllfläche (5) der Außenwand (3) und eine äußere Hüllfläche (6) der Innenwand (4), die der Außenwand (3) zugewandt ist, zusammen eine Eingrenzung (7) definieren, die dazu ausgelegt ist, feste Reaktionselemente oder einen starren Körper aus einem Reaktionselementmaterial zu enthalten.

2. Zylindrischer Strömungsverteiler nach Anspruch 1, wobei das erste Gerüst (22) ein erster Draht (26) ist, der spiralförmig um die periphere Außenfläche (29) der ersten Mehrzahl von sich in Längsrichtung erstreckenden Rippen (20) gewickelt ist.

3. Zylindrischer Strömungsverteiler nach Anspruch 1 oder 2, wobei die Spalte (21), die sich zwischen zwei benachbarten Rippen (20) erstrecken, eine entlang der inneren Hüllfläche der Eingrenzung (7) gemessene Breite W_{A} aufweisen, die kleiner als die Größe von festen Reaktionselementen oder des starren Körpers aus einem Reaktionselementmaterial, die in der Eingrenzung (7) enthalten sein sollen, ist.

4. Zylindrischer Strömungsverteiler nach einem der vorhergehenden Ansprüche, wobei jede Rippe (20) in der ersten Mehrzahl von Rippen (20) einen dreieckigen oder trapezförmigen Querschnitt aufweist, und wobei jede Rippe (20) an einer Innenfläche des ersten Gerüsts (22) über ihren Scheitel, falls der Querschnitt dreieckig ist, oder über ihre kürzeste parallele Kante, falls der Querschnitt trapezförmig ist, fest angebracht ist, wobei die sich zwischen zwei benachbarten Rippen (20) erstreckenden Spalte (21) gemessen entlang der inneren Hüllfläche (5) der Außenwand (3) kleiner sind als die sich zwischen zwei benachbarten Rippen (20) erstreckenden Spalte (27) gemessen entlang einer äußeren Hüllfläche (28) der Außenwand (3).

5. Zylindrischer Strömungsverteiler nach Anspruch 1, wobei die Spalte (33) zwischen jeder Windung des zweiten Drahts (31) eine in der axialen Richtung entlang der äußeren Hüllfläche (6) der Innenwand (4) gemessene Breite aufweisen, die kleiner als die Größe von festen Reaktionselementen oder des starren Körpers aus einem Reaktionselementmaterial ist, die in der Eingrenzung (7) enthalten sein sollen.

6. Zylindrischer Strömungsverteiler nach einem der vorhergehenden Ansprüche, wobei der zweite Draht (31) einen dreieckigen oder trapezförmigen Querschnitt aufweist, und wobei der zweite Draht (31) an der Außenfläche (32) des zweiten Gerüsts (30) über seinen Scheitel, falls der Querschnitt dreieckig ist, oder über seine kürzeste parallele Kante, falls der Querschnitt trapezförmig ist, fest angebracht ist, wobei die sich zwischen zwei benachbarten Windungen des zweiten Drahts (31) erstreckenden Spalte (33) gemessen entlang der äußeren Hüllfläche (6) der Innenwand (3), die der Außenwand (4) zugewandt ist, kleiner sind als sich zwischen zwei benachbarten Windungen des zweiten Drahts (31) erstreckende Spalte (35) gemessen entlang einer inneren Hüllfläche (36) der Innenwand (4).

7. Zylindrischer Strömungsverteiler nach einem der Ansprüche 4-6, wobei das zweite Gerüst (30) eine zweite Mehrzahl von sich axial erstreckenden Rippen (34) umfasst.

8. Reaktor zum Durchführen, mittels fester Reaktionselemente, einer biologischen oder chemischen Transformation oder eines physikalischen oder chemischen Einfangens aus oder Freisetzens von Agenzien in ein fluidförmiges Medium, wobei der Reaktor ein Gefäß (200) umfasst, in dem ein zylindrischer Strömungsverteiler (100) montiert wurde, wobei der zylindrische Strömungsverteiler (100) Folgendes umfasst:
eine obere Wand (1);
eine untere Wand (2), die eine mittige durchgehende Öffnung (13) umfasst;
eine Außenwand (3), die sich zwischen der oberen Wand (1) und der unteren Wand (2) erstreckt;
eine Innenwand (4); und
ein Mittel zum Drehen des zylindrischen Strömungsverteilers (100);
wobei die Außenwand (3) eine erste Mehrzahl von sich in Längsrichtung erstreckenden Rippen (20), die nebeneinander entlang einer Längsmittellinie CL des Strömungsverteilers angeordnet sind, wobei sich Spalte (21) in der Umfangsrichtung zwischen zwei benachbarten Rippen (20) erstrecken, und ein sich in Umfangsrichtung erstreckendes erstes Gerüst (22) umfasst, das eine periphere Außenfläche (29) der Mehrzahl von sich in Längsrichtung erstreckenden Rippen (20) umgibt und daran fest angebracht ist,
wobei die Innenwand (4) ein zweites Gerüst (30), das eine Erstreckung entlang einer axialen Richtung des Strömungsverteilers (100) aufweist, und eine Mehrzahl von aufeinanderfolgenden Windungen eines zweiten Drahts (31) umfasst, der an einer Außenfläche (32) des zweiten Gerüsts (30) derart angebracht ist, dass sich axial erstreckende Spalte (33) zwischen jeder Windung des zweiten Drahts (31) gebildet sind; und
wobei die obere Wand (1), die untere Wand (2), eine innere Hüllfläche (5) der Außenwand (3) und eine äußere Hüllfläche (6) der Innenwand (4), die der Außenwand (3) zugewandt ist, zusammen eine Eingrenzung (7) definieren, die dazu ausgelegt ist, feste Reaktionselemente oder einen starren Körper aus einem Reaktionselementmaterial zu enthalten.

9. Verwendung eines Reaktors nach Anspruch 8 zum Durchführen, mittels fester Reaktionselemente, einer biologischen oder chemischen Transformation oder eines physikalischen oder chemischen Einfangens aus oder Freisetzens von Agenzien in ein fluidförmiges Medium.

## Revendications

1. Distributeur d'écoulement cylindrique destiné à réaliser, au moyen d'éléments de réaction solides, une transformation biologique ou chimique ou un piégeage physique ou chimique à partir de, ou une libération d'agents dans, un milieu fluidique, le distributeur d'écoulement (100) comprenant :
une paroi supérieure (1) ;
une paroi inférieure (2) comprenant une ouverture traversante centrale (13) ;
une paroi extérieure (3) s'étendant entre la paroi supérieure (1) et la paroi inférieure (2) ; et
une paroi intérieure (4) ;
dans lequel la paroi extérieure (3) comprend une première pluralité de nervures s'étendant longitudinalement (20) disposées côte à côte le long d'une ligne centrale longitudinale CL du distributeur d'écoulement (100), des espaces (21) s'étendant dans la direction circonférentielle entre deux nervures adjacentes (20), et un premier échafaud s'étendant circonférentiellement (22) encerclant et étant fixé à demeure à une surface externe périphérique (29) de ladite pluralité de nervures s'étendant longitudinalement (20) ;
dans lequel la paroi intérieure (4) comprend un second échafaud (30) ayant une extension le long d'une direction axiale du distributeur d'écoulement (100), et une pluralité de spires consécutives d'un second fil (31) fixé à demeure sur une surface externe (32) dudit second échafaud (30), de sorte que des espaces s'étendant axialement (33) sont formés entre chaque spire dudit second fil (31) ; et
dans lequel la paroi supérieure (1), la paroi inférieure (2), une surface d'enveloppe intérieure (5) de la paroi extérieure (3) et une surface d'enveloppe extérieure (6) de la paroi intérieure (4) faisant face à la paroi extérieure (3) définissent conjointement un confinement (7) conçu pour contenir des éléments de réaction solides ou un corps rigide d'un matériau d'élément de réaction.

2. Distributeur d'écoulement cylindrique selon la revendication 1, dans lequel le premier échafaud (22) est un premier fil (26) en spirale enroulé autour de la surface externe périphérique (29) de ladite première pluralité de nervures s'étendant longitudinalement (20).

3. Distributeur d'écoulement cylindrique selon la revendication 1 ou 2, dans lequel les espaces (21) s'étendant entre deux nervures adjacentes (20) ont une largeur W_{A} telle que mesurée le long de la surface d'enveloppe intérieure dudit confinement (7) inférieure à la taille d'éléments de réaction solides ou du corps rigide d'un matériau d'élément de réaction destiné(s) à être contenu(s) dans le confinement (7).

4. Distributeur d'écoulement cylindrique selon l'une quelconque des revendications précédentes, dans lequel chaque nervure (20) de la première pluralité de nervures (20) a une section transversale triangulaire ou trapézoïdale, et dans lequel chaque nervure (20) est fixée à demeure à une surface intérieure du premier échafaud (22) par l'intermédiaire de son sommet dans le cas où la section transversale est triangulaire ou par l'intermédiaire de son bord parallèle le plus court dans le cas où la section transversale est trapézoïdale, grâce à quoi les espaces (21) s'étendant entre deux nervures adjacentes (20) tels que mesurés le long de la surface d'enveloppe intérieure (5) de ladite paroi extérieure (3) sont inférieurs aux espaces (27) s'étendant entre deux nervures adjacentes (20) tels que mesurés le long d'une surface d'enveloppe extérieure (28) de ladite paroi extérieure (3).

5. Distributeur d'écoulement cylindrique selon la revendication 1, dans lequel les espaces (33) entre chaque spire dudit second fil (31) ont une largeur telle que mesurée dans la direction axiale le long de la surface d'enveloppe extérieure (6) de ladite paroi intérieure (4) inférieure à la taille d'éléments de réaction solides ou du corps rigide d'un matériau d'élément de réaction destiné(s) à être contenu(s) dans le confinement (7).

6. Distributeur d'écoulement cylindrique selon l'une quelconque des revendications précédentes, dans lequel le second fil (31) a une section transversale triangulaire ou trapézoïdale, et dans lequel le second fil (31) est fixé à demeure sur la surface externe (32) du second échafaud (30) par l'intermédiaire de son sommet dans le cas où la section transversale est triangulaire ou par l'intermédiaire de son bord parallèle le plus court dans le cas où la section transversale est trapézoïdale, grâce à quoi les espaces (33) s'étendant entre deux spires adjacentes du second fil (31) tels que mesurés le long de la surface d'enveloppe extérieure (6) de ladite paroi intérieure (4) faisant face à la paroi extérieure (3) sont inférieurs aux espaces (35) s'étendant entre deux spires adjacentes du second fil (31) tels que mesurés le long d'une surface d'enveloppe intérieure (36) de ladite paroi intérieure (4).

7. Distributeur d'écoulement cylindrique selon l'une quelconque des revendications 4 à 6, dans lequel le second échafaud (30) comprend une seconde pluralité de nervures s'étendant axialement (34).

8. Réacteur destiné à réaliser, au moyen d'éléments de réaction solides, une transformation biologique ou chimique ou un piégeage physique ou chimique à partir de, ou une libération d'agents dans, un milieu fluidique, ledit réacteur comprenant une cuve (200) dans laquelle un distributeur d'écoulement cylindrique (100) a été monté, ledit distributeur d'écoulement cylindrique (100) comprenant :
une paroi supérieure (1) ;
une paroi inférieure (2) comprenant une ouverture traversante centrale (13) ;
une paroi extérieure (3) s'étendant entre la paroi supérieure (1) et la paroi inférieure (2) ;
une paroi intérieure (4) ; et
des moyens de rotation du distributeur d'écoulement cylindrique (100) ;
dans lequel la paroi extérieure (3) comprend une première pluralité de nervures s'étendant longitudinalement (20) disposées côte à côte le long d'une ligne centrale longitudinale CL du distributeur d'écoulement, des espaces (21) s'étendant dans la direction circonférentielle entre deux nervures adjacentes (20), et un premier échafaud s'étendant circonférentiellement (22) encerclant et étant fixé à demeure à une surface externe périphérique (29) de ladite pluralité de nervures s'étendant longitudinalement (20) ;
dans lequel la paroi intérieure (4) comprend un second échafaud (30) ayant une extension le long d'une direction axiale du distributeur d'écoulement (100), et une pluralité de spires consécutives d'un second fil (31) fixé à demeure sur une surface externe (32) dudit second échafaud (30), de sorte que des espaces s'étendant axialement (33) sont formés entre chaque spire dudit second fil (31) ; et
dans lequel la paroi supérieure (1), la paroi inférieure (2), une surface d'enveloppe intérieure (5) de la paroi extérieure (3) et une surface d'enveloppe extérieure (6) de la paroi intérieure (4) faisant face à la paroi extérieure (3) définissent conjointement un confinement (7) conçu pour contenir des éléments de réaction solides ou un corps rigide d'un matériau d'élément de réaction.

9. Utilisation d'un réacteur selon la revendication 8 pour réaliser au moyen d'éléments de réaction solides, une transformation biologique ou chimique ou un piégeage physique ou chimique à partir de, ou une libération d'agents dans, un milieu fluidique.
